# EUROPEAN PATENT APPLICATION

(11) **EP 3 875 453 A1**
(43) Date of publication of application: **08.09.2021**
(21) Application number: 21160054.9
(22) Date of filing: 01.03.2021
(51) Int. Cl.: C07D 257/00

(54) **METHOD FOR MANUFACTURING CALCOBUTROL**

(30) Priority: 06.03.2020 KR 20200028549
(71) Applicant: Enzychem Lifesciences Corporation, Chungcheongbuk-do 27159 (KR)
(72) Inventor: LEE, Jong Soo, 26457 Wonju-si (KR); YUN, Dae Myoung, 26432 Wonju-si (KR); LEE, Byuong Woo, 27109 Jecheon-si (KR)
(74) Representative: Henkel & Partner mbB

(57) **Abstract**

It is disclosed a method for preparing calcobutrol, an excipient for preparing gadobutrol injections used as MRI contrast agents, the present invention provides a method for producing calcobutrol comprising: reacting lithium chloride and 4,4-dimethyl-3,5,8-trioxabicyclo[5,1,0]octane with 1,4,7,10-tetraazacyclododecane represented by the following Formula 1 to obtain a calcobutrol intermediate represented by the following Formula 2; reacting the calcobutrol intermediate represented by Formula 2 with chloroacetic acid to obtain butrol represented by the following Formula 3; and reacting butrol represented by Formula 3 with calcium ion to obtain calcobutrol represented by Formula 4 below.

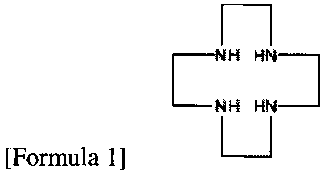

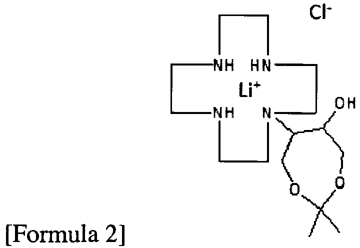

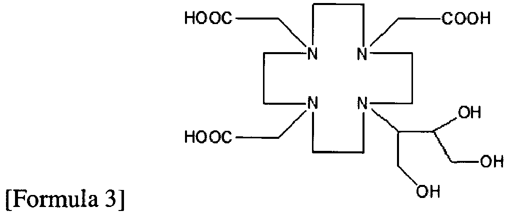

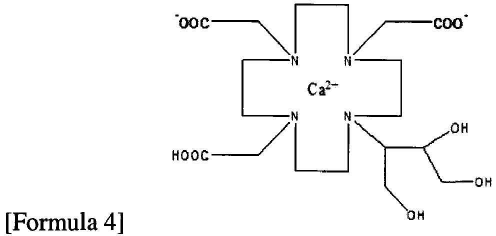

## Description

### CROSS-REFERENCE TO RELATED APPLICATION

This application claims benefit of priority to Korean Patent Application No. 10-2020-0028549 filed on March 06, 2020, the entire content of which is incorporated herein by reference.

### FIELD OF THE DISCLOSURE

The present invention relates to a method for preparing calcobutrol, and more particularly, to a method for manufacturing calcobutrol used in preparing an MRI contrast agent gadobutrol injection.

### BACKGROUND

Gadobutrol is marketed under the trade name of Gadovist or Gadavist in the field of contrast agent containing gadolinium, all over the world.

In the case of such a gadolinium-containing contrast agent, it has been found to be advantageous to apply an excess of the complex-forming ligand in the form of a calcium complex. Here, the role of the calcium complex is to prevent the release of free gadolinium from gadobutrol after preparation, thereby solving the safety problem for nephrotic systemic fibrosis (NSF) caused by the toxicity of gadolinium cations.

The synthesis method of calcobutrol is described in detail in the literature (Inorg. Chem. 1997, 36, 6086-6093). However, although a material having a purity of 90 to 95% can be obtained by the method of the above document, this does not reach the purity required for formulation.

Further purification of 2,2,2-(10-1,3,4-trihydroxybutan-2-yl)-1,4,7,10-tetraazacyclododecane-1,4,7-triyl)triacetic acid (hereinafter, "butrol") is not easy to purify with an ion exchange resin by the zwitterionic of butrol, and crystallization through pH adjustment is not performed, so purification by crystallization is also not possible.

Gadobutrol, a neutral gadolinium complex, can be obtained with high purity (> 99.6%) through crystallization after the reaction is terminated and purified by an ion exchange column, but calcobutrol has a problem that purification is not easy due to the remaining acid functional groups. Therefore, it is known that the method of preparing calcobutrol directly from butrol is not suitable in terms of purity.

According to Bayer's registered Korean patent No. 10-1057939, it is known that the gadobutrol, which has already been obtained, is selected as a starting material and decomplexed, and free gadolinium is removed to prepare high purity butrol, and then complexed with calcium to prepare calcobutrol, in order to prepare calcobutrol of high purity. However, in the case of oxalic acid, which is used for decomplexation in the above method, it is toxic and thus has limitations in its use. In addition, through the process of adsorbing and desorbing butrol on the cation exchange resin, other impurities are removed to obtain butrol of high purity, and it reacts with calcium ion to obtain calcobutrol. These series of processes are not economical, and the process is also complicated.

According to registered Korean patent No. 10-1693400 of ST Pharm Co., Ltd., it is known that 3-(1,4,7,10-tetraazacyclododecan-1-yl)butane-1,2,4-triol tetrahydrochloride, which is gadobutrol intermediate is used as a starting material, and introduced in tert-butylbromoacetate. After undergoing a deprotection process, high purity butrol is obtained through resin purification, and calcobutrol is obtained by reacting with calcium ion. However, in case of using the tert-butyl bromoacetate in the above method, it is harmful to the human body and the price is very high. Also, since the deprotector process is added, it is not economical, and the process becomes complicated.

Therefore, there is a need for development of a method for producing calcobutrol that it is less harmful to the human body and eco-friendly, and economical by excluding resin purification and deprotection processes, and high purity through a simplified process.

### SUMMARY OF THE INVENTION

### TECHNICAL OBJECTS

Accordingly, an object of the present invention is to have a high purity by purifying by a nanofilter (nanofiltration) process using cyclone, which is a cheaper raw material than the conventional one, as a starting material. And the overall preparing process is simple, and calcobutrol can be produced economically.

Another object of the present invention is economical compared to the generally known process using gadobutrol as a starting material. The present invention does not use materials harmful to the human body such as oxalic acid, and thus provides a method for preparing calcobutrol more safely.

### TECHNICAL SOLUTION

In order to achieve the above object, the present invention provides a method for producing calcobutrol comprising: reacting lithium chloride and 4,4-dimethyl-3,5,8-trioxabicyclo[5,1,0]octane with 1,4,7,10-tetraazacyclododecane represented by the following Formula 1 to obtain a calcobutrol intermediate represented by the following Formula 2; reacting the calcobutrol intermediate represented by Formula 2 with chloroacetic acid to obtain butrol represented by the following Formula 3; and reacting butrol represented by Formula 3 with calcium ion to obtain calcobutrol represented by Formula 4 below.

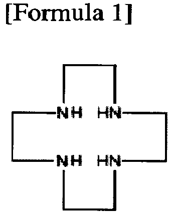

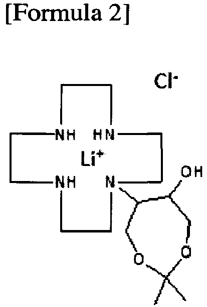

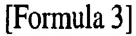

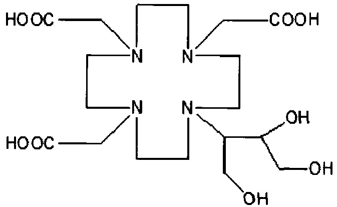

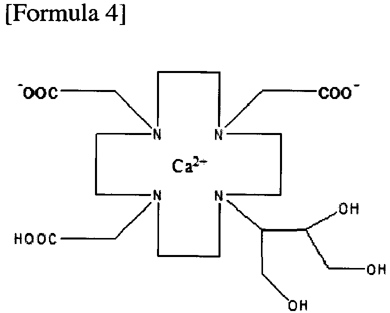

### EFFECTS OF THE INVENTION

As described above, the method for preparing calcobutrol according to the present invention is economical by using cyclone, which is an inexpensive raw material, as a starting material. It can be prepared more safely because it does not use materials harmful to the human body such as oxalic acid. In addition, high purity calcobutrol can be prepared by purification by a nanofilter (nanofiltration) process.

### DETAILED DESCRIPTION OF THE PREFERRED EMBODIMENTS

Hereinafter, the present invention will be described in more detail.

The present invention discloses a method for preparing calcobutrol, as shown in Scheme 1 below, for preparing calcobutrol using cyclone as a starting material.

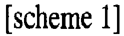

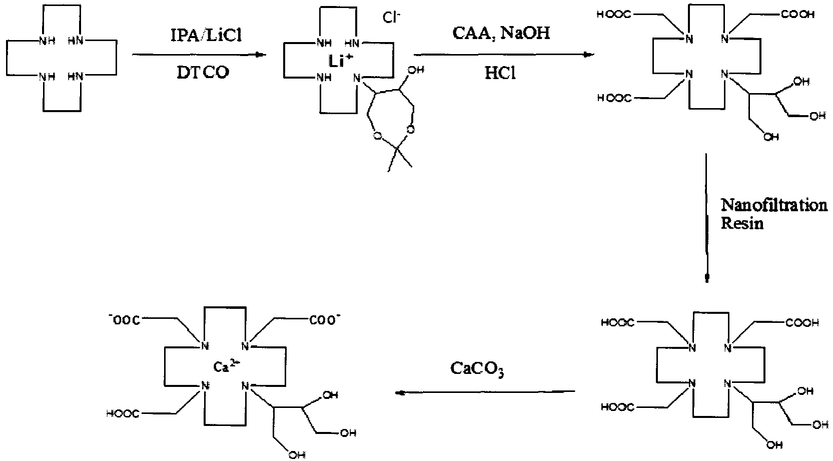

After preparing an intermediate using lithium chloride and DTCO in the cyclone, chloroacetic acid, sodium hydroxide, and hydrochloric acid are used, and then butrol of high purity is obtained using a nanofilter and an ion exchange resin. Wherein calcium carbonate is used to provide calcium ion and crystallize to prepare high-purity calcobutrol in high yield.

Specifically, in order to prepare calcobutrol according to the present invention, first, a cyclene-lithium chloride complex is prepared by reacting 1,4,7,10-tetraazacyclododecane (hereinafter referred to as "cyclene") represented by the following Formula 1 as a starting material. Then, 4,4-dimethyl-3,5,8-trioxabicyclo[5,1,0]octane is reacted to obtain N-(6-hydroxy-2,2-dimethyl-1,3-dioxyphen-5-yl)-1,4,7,10-tetraazacyclododecane-lithium chloride complex (hereinafter referred to as "calcobutrol intermediate") represented by the following Formula 2.

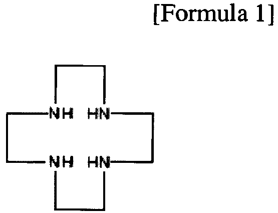

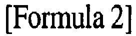

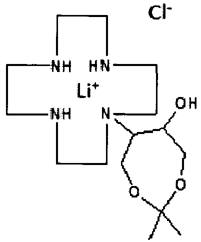

The reaction may be carried out in a solvent such as isopropyl alcohol, purified water, methanol, and ethanol, and the reaction temperature is generally 75 to 100°C. The amount of lithium chloride used is 0.8 to 1.5 equivalents, preferably 1.05 to 1.2 equivalents, with respect to 1 equivalent of cyclene. If the amount of lithium chloride is too small, there is a problem that the purity is lowered, and if too much, there is a problem that the amount of chloride increases.

The amount of the 4,4-dimethyl-3.,5,8-trioxabicyclo[5,1,0]octane used is 1.0 to 1.5 equivalents, preferably 1.2 to 1.4 equivalents based on the cyclene-lithium chloride complex. If the amount of the 4,4-dimethyl-3,5,8-trioxabicyclo[5,1,0]octane is too small, there is a problem of reducing the yield due to unreacted substances, and if too much, there is a problem of reducing the purity and yield by pyrolysis by-products.

Next, the calcobutrol intermediate represented by Formula 2 and chloroacetic acid are reacted to obtain 2,2,2-((10-1,3,4-trihydroxybutan-2-yl)-1,4,7,10-tetraazacyclododecane-1,4,7-triyl)tri acetic acid (hereinafter, referred to as "butrol") represented by Formula 3.

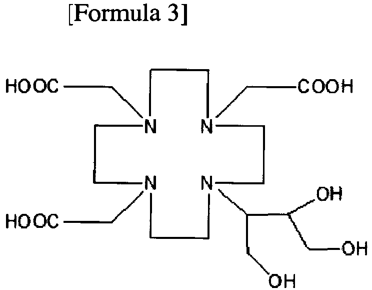

The reaction may be carried out in an alkaline water solvent. For example, as a solvent for the reaction, sodium hydroxide (NaOH) is added dropwise to water to form an alkaline medium having a pH of 8 to 12, preferably 9 to 10. The reaction can generally be carried out at a temperature of 75 to 85 °C. In the reaction, the amount of chloroacetic acid used is 3.0 to 4.5 equivalents, preferably 3.4 to 4.0 equivalents, based on the calcobutrol intermediate represented by Formula 2. Wherein, if the amount of the chloroacetic acid is too small, there is a problem in decreasing the yield and purity by the unreacted products, and if it is too large, there is a problem in removing the unreacted products and the decomposition products.

The reaction product is concentrated and filtered under acidic conditions, specifically it is purified using a nanofilter. The nanofilter system may separate and purify inorganic materials having a salt and other molecular weight through an organic layer, as a reverse osmosis device designed to filter or concentrate substances having a molar mass of 200 to 300 Dalton or more in the spiral type of an organic layer. The filtrate purified by the nanofilter may be further purified by an ion exchange resin process, and the ion exchange resin may be used in a cascade manner as a cation exchange resin column and an anion exchange resin column. When the reaction product including the salt generated from the reaction is filtered again, the remaining decomplexing agents and by-products can be removed. When the purified filtrate is concentrated, butrol having a purity of 99.7% or more can be obtained.

Next, the butrol represented by Formula 3 and calcium ion are reacted and crystallized to obtain 2,2,2-(10-1,3,4-trihydroxybutan-2-yl)-1,4,7,10-tetraazacyclododecane-1,4,7-triyl)triacetic acid calcium complex(hereinafter, calcobutrol) represented by Formula 4.

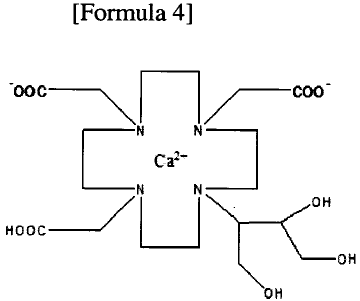

The reaction uses purified water. The calcium ion source may include calcium carbonate, calcium hydroxide, calcium chloride, and the like, and calcium carbonate is preferably used. The amount of the calcium ion source used is 0.95 to 1.05 equivalents, preferably 1.0 equivalent, based on (with respect to) 1.0 equivalent of butrol. Wherein, if the amount of the calcium source is too small, the complex is less formed, resulting in a decrease in yield, and if it is too large, a complex having a content ratio of butrol and calcium of 2:3 may be formed.

The reaction temperature is generally carried out at a temperature of 80 to 90°C, and if the temperature is too low, there is a decrease in the yield due to unreacted products, and if the temperature is too high, a problem may occur in the quality of the product. In addition, the reaction time of the butrol and calcium ion is 1 hour to 2 hours, and if the reaction time is short, problems may occur during crystallization and yield reduction by unreacted products, and if it is too long, quality problems such as the formation of related substances may occur.

After the reaction product is concentrated, it can be dissolved in purified water and crystallized and isolated with a crystallization solvent. The crystallization solvent may be used organic solvents such as methanol, ethanol, isopropanol, and acetone, and acetone may be used preferably. Specifically, the mother liquor may be crystallized in purified water-acetone conditions generally at 45 to 55°C. The crystallized mixture is dried to obtain calcobutrol having a purity of 99.0% or more.

Hereinafter, the present invention will be described in more detail through examples, but the present invention is not limited by the following examples.

Example 1 : Preparation of butrol represented by Formula 3

1,4,7,10-tetraazacyclododecane (100g, 1eq), lithium chloride (29.55g, 1.14 eq), 4,4-dimethyl-3,5,8-trioxabicyclo[5.1.0] octane (83.75g, 1eq) and isopropyl alcohol (219.6g, 2.2vol) were added to the reactor and the temperature was raised to 85 to 95°C to proceed the reaction. After completion of the reaction, 830.49 g of methyl tert-butyl ether was added, stirred at 20 to 25°C for 1 hour, and filtered. Then, it was washed with 79.56 g of methyl tert-butyl ether. The filtrate was concentrated under decompressed pressure, and then 642.5 g of purified water was added. The temperature was raised to 50 °C and chloroacetic acid (139.1g, 3.7eq) was added. 40% NaOH was added dropwise, and heated and stirred at 75 to 85°C, while maintaining pH 9 to 10, and then the reaction was terminated. It was added 14.83g of hydrochloric acid, and then concentrated under decompressed pressure. Salt was filtered by adding 128.5 g of methanol, and a nano filter was performed. Thereafter, 650 g of each of weak basic resin and weak acidic resin were put in a resin column, the reaction solution was treated 10 times, and then concentrated under decompressed pressure to obtain 100 g of a clear liquid butrol (yield 38.26 wt%, purity 99.8%, moisture 11.2%).

Example 2-1: Preparation of calcobutrol(solid) using acetone.

50.0 g of butrol prepared in Example 1, 9.87g of calcium carbonate, and 200 ml of purified water were added to the reactor. Then, the temperature was raised to 75°C, stirred for 3 hours, cooled and filtered. It was concentrated, and then added 50ml of purified water. The temperature was raised to 40 °C, and added 600ml of acetone. When crystals were formed, the mixture was cooled to 20°C, and the resulting crystals were filtered and dried to obtain 46.12 g of a white calcobutrol solid (yield 85.0%).

Example 2-2: Preparation of calcobutrol(solid) using anhydrous ethanol.

50.0 g of butrol prepared in Example 1, 9.87g of calcium carbonate, and 200 ml of purified water were added to the reactor. The temperature was raised to 75°C, stirred for 3 hours, cooled and filtered. It was concentrated, and the temperature was raised to 60°C, and then added 200ml of anhydrous ethanol. When a solid was formed, it was concentrated and anhydrous ethanol was added until the moisture was 3%, followed by further concentration. When the moisture content reached 3%, the resulting crystal was cooled to 20°C and filtered. It was dried to obtain 49.37 g (yield 91.0%) of a white calcobutrol solid.

## Claims

1. A method for producing calcobutrol comprising:
reacting lithium chloride and 4,4-dimethyl-3,5,8-trioxabicyclo[5,1,0]octane with 1,4,7,10-tetraazacyclododecane represented by the following Formula 1 to obtain a calcobutrol intermediate represented by the following Formula 2; 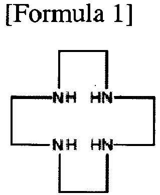 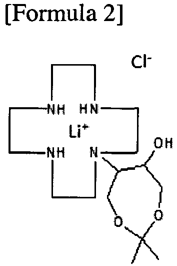
reacting the calcobutrol intermediate represented by Formula 2 with chloroacetic acid to obtain butrol represented by the following Formula 3; and 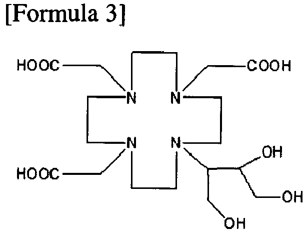
reacting butrol represented by Formula 3 with calcium ion to obtain calcobutrol represented by Formula 4 below.

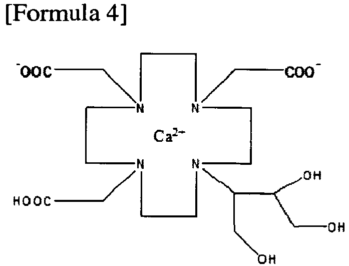

2. The method of claim 1, wherein the pH is 8 to 12 when preparing the butrol.

3. The method of claim 1, wherein the butrol is purified using nanofilter.

4. The method of claim 1, wherein the calcium ion is selected from the group consisting of calcium carbonate, calcium hydroxide, calcium chloride, and mixtures therof, and the content of calcium ion is 0.95 to 1.05 equivalents based on the butrol.

5. The method of claim 1, wherein the amount of chloroacetic acid used is 3.0 to 4.5 equivalents based on the calcobutrol immediate.

6. The method of claim 1, wherein the amount of 4,4-dimethyl-3,5,8-trioxabicyclo[5,1,0]octane used is 1.0 to 1.5 equivalents based on the cyclen-lithium chloride complex.

7. The method of claim 1, wherein it is crystallized using a crystallization solvent selected from the group consisting of methanol, ethanol, isopropanol, and acetone, in the step of obtaining calcobutrol.
